# FASCICULE DE BREVET EUROPEEN

(11) **EP 1 058 540 B1**
(45) Date de publication et mention de la délivrance du brevet: **31.10.2001**
(21) Numéro de dépôt: 99904939.8
(22) Date de dépôt: 24.02.1999
(51) Int. Cl.: A61K 9/16

(54) **PELLET ORAL A LIBERATION IMMEDIATE COMPRENANT DES GLYCERIDES POLYGLYCOLYSES, PROCEDE DE FABRICATION**
POLYGLYKOLISIERTE GLYCERIDE ENTHALTENDE RASCH-FREISETZENDE PELLETFORMULIERUNG, VERFAHREN ZUR HERSTELLUNG
ORAL PELLET WITH IMMEDIATE RELEASE COMPRISING GLYCERIDES SUBJECTED TO POLYGLYCOLYSIS, METHOD FOR MAKING SAME

(30) Priorité: 04.03.1998 FR 9802855
(43) Date de publication de la demande: 13.12.2000
(73) Titulaire: GATTEFOSSE S.A., 69800 Saint Priest (FR)
(72) Inventeur: BARTHELEMY, Philippe, F-69780 Myons (FR); FARAH, Nabil, F-69007 Lyon (FR); ROUSSIN, Pascale, F-69002 Lyon (FR)
(74) Mandataire: Vuillermoz, Bruno
(86) Numéro de dépôt international: FR9900406
(87) Numéro de publication internationale: WO9944589

(56) Documents cités:
- WO-A-94/23700
- WO-A-98/10762
- WO-A-99/21534
- US-A- 4 786 495
- US-A- 4 869 900
- A.ESQUISABEL, A. SAN VINCENTE, M. IGARTUA, R.M.HERN NDEZ, A.R. GASCON, M.B. CALVO AND J.L. PEDRAZ: "Influence of melting point and hydrophilic/lipophilic balance on the release of salbutamol sulfate from lipid matrices" STP PHARMA SCIENCES, vol. 6, no. 5, 1996, pages 365-69, XP002087329

## Description

L'invention se rapporte à une forme galénique particulière dénommée « pellet » à libération immédiate de substance active destinée à être ingérée et apte à améliorer la biodisponibilité de la substance active. Elle se rapporte également au procédé de fabrication de cette pellet.

Le Demandeur a développé des compositions administrables par voie orale, aptes à améliorer la biodisponibilité du principe actif. Ces compositions sont connues sous le terme SMEDDS (Self Microemulsifying Drug Delivery System).

Les SMEDDS sont plus particulièrement décrits dans le document du Demandeur EP-A-0 670 715. Ils sont constitués d'un mélange de un ou plusieurs principes actifs avec une phase lipophile, un agent tensioactif, un agent cotensioactif, dont les caractéristiques sont déterminées de sorte à ce que le produit final soit apte à former une microémulsion au contact d'un volume donné de liquide physiologique.

Certains produits commercialisés par le Demandeur, constitués d'acides gras saturés et/ou insaturés et d'esters de ces acides gras, peuvent être utilisés en tant que phase lipophile, agent tensioactif et agent co-tensioactif, telle que par exemple l'association respectivement de GELUCIRE® 44/14, LABRAFAC® CM10 et LAUROGLYCOL® décrits dans les exemples 1 et 2 du document suscité.

En pratique, le procédé de fabrication de ces compositions consiste :
- tout d'abord, à liquéfier si nécessaire, en chauffant, la phase lipophile (par exemple GELUCIRE® 44/14 solide à température ambiante) ;
- puis à mélanger ladite phase lipophile avec les proportions indiquées d'agent tensioactif (par exemple LABRAFAC® CM10) et d'agent cotensioactif (LAUROGLYCOL® )
- puis, sous agitation, à disperser la ou les substances actives dans le mélange obtenu.

Le mélange obtenu est directement injecté à l'état liquide dans des gélules ou des capsules molles et ce, le cas échéant, avant solidification à température ambiante.

Toutefois, cette méthode de conditionnement présente un certain nombre d'inconvénients puisque le remplissage des gélules par une formule de type SMEDDS à l'état liquide nécessite un équipement particulier supplémentaire et onéreux, dont ne disposent pas toujours les fabricants de produits finis. Il faut en effet disposer d'un système réservoir double enveloppe-agitation, d'un système de pompe à injection de précision. En outre, la fabrication de capsules nécessite également un matériel spécifique constitué d'une machine à banderoller les capsules de gélatine, ce type de conditionnement pouvant présenter par ailleurs, un risque de fuites du SMEDDS en dehors de la capsule. Il s'ensuit que la fabrication de ces capsules doit être sous-traitée par des façonniers spécialistes.

Pour augmenter la biodisponibilité du principe actif et plus particulièrement celle du Coenzyme Q₁₀, on a proposé dans le document US-A-4 869 900, une composition comprenant outre le Coenzyme Q₁₀ dans une proportion de 2 à 17%, de la lécithine, utilisée en tant qu'agent solubilisant, dans une proportion de 50 à 70% et du GELUCIRE® 50/13 ou 44/14, utilisés exclusivement an tant qu'agent tensioactif (c'est à dire émulsifiant), dans une proportion comprise entre 20 et 48%. En pratique, le Coenzyme Q₁₀ est mis au contact de l'agent tensio-actif à 60°C puis le produit obtenu est mélangé, sous agitation, avec la lécithine. Le mélange fluide obtenu est ensuite adsorbé sur un matériau inerte du type silice colloïdale. Les granulés résultants sont alors mis en sachet ou sont soumis à une étape de compression en présence d'additifs usuels de sorte à obtenir des comprimés. Il est en outre observé (exemple 8 et colonne 2, ligne 50) que la biodisponibilité du mélange Coenzyme Q₁₀ / lécithine et celle du mélange Coenzyme Q₁₀ / GELUCIRE® est très faible tandis que celle du mélange Coenzyme Q₁₀ / lécithine / GELUCIRE® est accrue, ce qui implique que l'association lécithine / GELUCIRE® exerce un effet synergique vis à vis de l'amélioration de la biodisponibilité.

On a également proposé, dans le document WO 94/23700, une composition pharmaceutique solide, sous forme de pellets, à libération immédiate de principe actif faiblement soluble, fabriquées comme suit. Le principe actif est tout d'abord dissous dans une phase liquide constituée au choix d'huiles, de solvants polaires, de corps gras ou d'agents tensio-actifs ioniques ou non ioniques (voir page 3, lignes 1 à 10). Le produit obtenu est ensuite aggloméré sur des particules de cellulose microcristalline par granulation par voie humide nécessitant de l'eau. Les particules agglomérées alors obtenues sont ensuite soumises à deux étapes de fabrication supplémentaires respectivement, une première étape d'extrusion dans un extrudeur et une seconde étape de sphéronisation dans un sphéronisateur. En d'autres termes, les pellets sont obtenues par un procédé long, en plusieurs étapes et nécessitant la présence d'eau, laquelle peut avoir un effet négatif sur la stabilité du principe actif, si ce dernier est sensible aux phénomènes d'oxydation.

On a par ailleurs décrit, dans le document WO 93/18753, un procédé de fabrication de pellets à libération prolongée de substance active par pelletisation par fusion encore désigné par le terme « melt pelletization » consistant, en une seule étape, à agglomérer, densifier et sphéroniser des microparticules. Dans le procédé décrit, l'agent liant est constitué d'une substance insoluble dans l'eau de type cire et dont le point de fusion est supérieur à 40°C.

Le problème que se propose de résoudre l'invention est donc de fournir une composition solide, notamment sous forme de pellets à libération immédiate de principe actif comprenant un nombre de constituants le plus faible possible, tout en améliorant la biodisponibilité relative. La biodisponibilité relative est la biodisponibilité obtenue par la voie orale par rapport à la biodisponibilité obtenue par la voie injectable.

Un autre problème de l'invention est de fournir une formulation du type SMEDDS qui qui puisse être conditionnée autrement qu'à l'état liquide sans nécessiter les équipements spécifiques et onéreux décrits préalablement.

Un autre but de l'invention est de proposer une composition du type SMEDDS qui puisse être mise en forme et conditionnée non seulement sous forme de gélules mais également sous d'autres formes galéniques classiques.

S'agissant du procédé de fabrication des pellets, le problème que se propose de résoudre l'invention est de développer un procédé nécessitant un nombre d'étapes minimum.

De même, et en liaison avec le produit obtenu, un autre problème que se propose de résoudre l'invention consiste à réduire le nombre de constituants nécessaires à la fabrication.

Pour résoudre le problème de fournir une composition solide, à libération immédiate de principe actif comprenant un nombre de constituants le plus faible possible, tout en présentant une biodisponibilité améliorée. l'invention propose une pellet à libération immédiate de substance active destinée à être ingérée, ladite pellet comprenant au moins une substance active, un agent liant et un agent diluant, caractérisée en ce que l'agent liant :
- comprend un mélange de glycérides polyglycolysés dont les acides gras comprennent au moins 8 atomes de carbone,
- présente un point de fusion supérieur ou égal à 37°C,
- présente une balance hydrophile / lipophile supérieure ou égale à 10,
ledit agent liant étant apte à améliorer la biodisponibilité de la substance active.

Dans la suite de la description et dans les revendications, on désigne par le terme « pellets », des formes solides agglomérées multiparticulaires de taille variant entre 0,5 et 5 millimètres, dont la surface est considérée comme sphérique, et l'état de surface homogène.

De même, par l'expression «glycérides polyglycolysés, dont les acides gras comprennent au moins 8 atomes de carbone », on désigne le produit de la réaction entre un polyethylène glycol de poids moléculaire compris entre 200 et 1500 et une huile de départ, ladite huile consistant en un mélange de triglycérides avec des acides gras choisis dans le groupe comprenant l'acide caprylique, caprique, myristique, palmitique, stéarique oléique, et linoleique, seuls ou en mélange.

En d'autres termes, le produit résultant de cette réaction est un mélange de mono-, di- et triglycérides et de mono- et diester de polyéthylèneglycol (PEG), de poids moléculaire compris entre 200 et 1500, éventuellement de glycérol et de PEG libre.

Le Demandeur a en effet constaté que les glycérides polyglycolysés comprenant au moins 8 atomes de carbone sélectionnés étaient non seulement susceptibles d'agir en tant qu'agent liant, en liant et en enrobant l'ensemble des constituants assurant ainsi la cohésion des particules entre elles, mais conférait également aux pellets finales une biodisponibilité améliorée.

En outre, les pellets de l'invention comprennent seulement trois constituants au minimum, (substance active, agent liant et agent diluant) contrairement à l'art antérieur où la présence d'une part, d'un agent solubilisant à part entière, et d'autre part d'un solvant du type eau, permettant d'agglomérer les particules par granulation par voie humide, était indispensable.

Avantageusement, l'agent liant comprend un mélange de glycérides polyglycolysés saturés dont les acides gras comprennent de 8 à 18 atomes de carbones (C₈-C₁₈), présente un point de fusion égal à 44°C et une balance hydrophile / lipophile égale à 14.

Par « C₈-C₁₈ », on désigne des mélanges en proportions significatives et variables des acides caprylique (C₈), caprique (C₁₀), laurique (C₁₂), myristique (C₁₄), palmitique (C₁₆) et stéarique (C₁₈), lorsque ces acides sont saturés et les acides insaturés correspondants en (C₈-C₁₈). Les proportions de ces acides gras peuvent varier en fonctions des huiles de départ.

De préférence, le mélange suscité comprend de 15 à 40% en poids d'esters de glycérol, le complément à 100% étant constitué d'esters de PEG.

Selon une autre forme de réalisation, l'agent liant comprend un mélange de glycérides polyglycolysés dont les acides gras comprennent 22 atomes de carbone (acide béhénique) seul ou en combinaison avec des acides gras en C₈-C₁₈ et ce, afin d'ajuster les propriétés filmogénes et liante du mélange ainsi qu'adapter les caractéristiques de libération recherchées pour la substance active mise en oeuvre.

En outre, ces pellets présentent l'avantage de pouvoir être incorporés tels quels dans les gélules, autrement dit sans phase de liquéfaction préalable, ne nécessitant donc pas d'équipement spécifique mais un simple matériel permettant de mettre en oeuvre des formes solides multiparticulaires.

Selon une autre caractéristique de l'invention, la concentration d'agent liant représente entre 10 et 40% en poids de la pellet, avantageusement de 15% à 20% en poids.

Pour une concentration inférieure à 10 %, on observe un manque de cohésion des pellets et une grande hétérogénéité de taille.

Pour une concentration supérieure à 40 %, on observe une agglomération des pellets formant des blocs compacts et souples.

Pour résoudre le problème d'obtenir une formulation du type SMEDDS® , l'agent liant pourra comprendre en outre un additif choisi dans le groupe comprenant le polyéthylène glycol, les esters de glycérol, les esters de polyéthylène glycol, les esters de propylène glycol, les esters de polyglycérol avec des acides gras saturés et /ou insaturés, seuls en mélange.

En effet, le choix de l'additif permet d'ajuster à la fois le point de fusion et la balance hydrophile / lipophile de l'agent liant, mais également d'améliorer son affinité avec la substance active.

Pour optimiser la tension de surface entre l'agent diluant et l'agent liant, on utilise un agent diluant choisi parmi les excipients traditionnels en pharmacie, soluble ou insoluble.

Avantageusement, l'agent diluant est choisi dans le groupe comprenant le diphosphate de calcium, le lactose et autres polyols, les esters de cellulose et les dérivés de silice.

Pour favoriser l'homogénéité du mélange de chacun des constituants et permettre la libération uniforme de la substance active, l'agent diluant se présente sous forme micronisée de taille comprise entre 1 et 300 micromètres, avantageusement entre 5 et 60 micromètres.

Pour une taille inférieure à 1 micromètre, on observe une mise en oeuvre délicate de la poudre due à sa légèreté et à son volume apparent élevé.

Pour une taille supérieure à 300 micromètres, on observe une hétérogénéité de la taille des pellets.

De même, avantageusement, la substance active se présente sous forme micronisée de taille comprise entre 1 et 300 micromètres, avantageusement entre 5 et 60 micromètres.

Comme déjà dit, un autre problème que se propose de résoudre l'invention est de développer un procédé nécessitant un nombre d'étapes minimum.

Pour ce faire, le procédé pour la fabrication de pellets de l'invention, se caractérise en ce que :
- tout d'abord, on introduit la substance active, l'agent liant et l'agent diluant dans un mélangeur muni d'un système d'agitation planétaire et de chauffage,
- puis, on agite tout en chauffant les constituants jusqu'à atteindre une température proche du point de fusion de l'agent liant,
- on poursuit l'agitation du mélange obtenu jusqu'à dispersion homogène de l'agent diluant et de la substance active dans l'agent liant,
- on maintient l'agitation et la température afin de permettre l'agglomération, la densification et la sphéronisation des particules formées jusqu'à l'obtention de pellets individualisées,
- toujours sous agitation et à une température sensiblement égale à celle du point de fusion de l'agent liant, on évacue les pellets individualisées en dehors du mélangeur,
- enfin, on récupère les pellets et on les refroidit à une température permettant de solidifier et de maintenir les pellets individualisées, séparées les unes des autres.

On a en effet constaté que le refroidissement des pellets à l'extérieur de la machine permettait d'éviter les phénomènes de collage des pellets contrairement à d'autres conditions de refroidissement.

Avantageusement, les pellets sont refroidis à une température comprise entre 4 et 30°C.

Selon un premier mode de réalisation du procédé de l'invention, le refroidissement des pellets est effectué sur des plateaux.

Selon un second mode de réalisation de l'invention, le refroidissement des pellets est effectué au moyen d'un lit d'air fluidisé.

Par ailleurs et selon une autre caractéristique, l'agitation est comprise entre 300 et 1000 tours par minute.

Pour une agitation inférieure à 300 tours par minute, on ne parvient pas à obtenir un mélange homogène.

Pour une agitation supérieure à 1000 tours, on observe un réchauffement trop important du mélange empêchant la formation des pellets.

Par ailleurs, le procédé de l'invention permet de réaliser des mises en forme variées.

En effet, les pellets refroidis peuvent être incorporés dans des gélules.

De même, ils peuvent être incorporés dans des sachets.

En d'autres termes, la sélection de l'agent liant permet, par ce procédé de préparation, de mettre en oeuvre des formulations présentant une biodisponibilité améliorée non pas sous forme liquide mais sous forme solide et ainsi de varier les mises en forme. De plus et comme déjà dit, les mises en forme peuvent être effectuées à l'aide d'un matériel classique de l'industrie pharmaceutique pour formes solides multiparticulaires en une seule opération.

L'invention concerne également les pellets susceptibles d'être obtenues par le procédé décrit ci-avant.

L'invention et les avantages qui en découlent ressortiront mieux des exemples de réalisation suivants à l'appui des figures annexées dans lesquelles :
- la figure 1 représente la distribution de taille de pellets fabriqués conformément à l'invention pour des temps d'agitation de 35 minutes (A), 32 minutes (B) et 29 minutes ( C ) en utilisant en temps qu'agent liant le Gélucire ® 44/14;
- la figure 2 représente la distribution de taille de pellets fabriqués conformément à l'invention en utilisant en temps qu'agent liant le Gélucire ® 50/13.

Dans les exemples suivants, les pellets ont été fabriquées dans un mélangeur grande vitesse du type VG 25 de Glatt.

### Exemple 1

On introduit dans le mélangeur chacun des constituants, à savoir respectivement :
. substance active (indometacine) sous forme micronisée: 4 %
. agent liant (Gélucire ® 44/14): 16 %
. agent diluant (lactose) sous forme micronisée: 80 %

La micronisation est effectuée à une taille d'environ 60 micromètres

On agite les constituants du mélange à raison de 600 tours/minute tout en chauffant la double enveloppe à une température sensiblement égale au point de fusion du GELUCIRE, à savoir à 44°C. La température de fusion est obtenue au bout de cinq minutes.

On fait ensuite varier le temps d'agitation comme ci-après :
A : 35 minutes
B : 32 minutes
C : 29 minutes

Une fois que l'agglomération, la densification et la sphéronisation des particules formées est obtenue, toujours sous agitation, on décharge les pellets au moyen de vannes de sortie.

En d'autre termes, le refroidissement des pellets n'a pas lieu dans la machine comme c'était le cas antérieurement, mais à l'extérieur de la machine. En a en effet constaté que cette technique permettait d'éviter le collage des pellets sur les parois de la machine.

On dispose les pellets individualisés sur des plateaux pour permettre leur refroidissement entre 19 et 25°C.

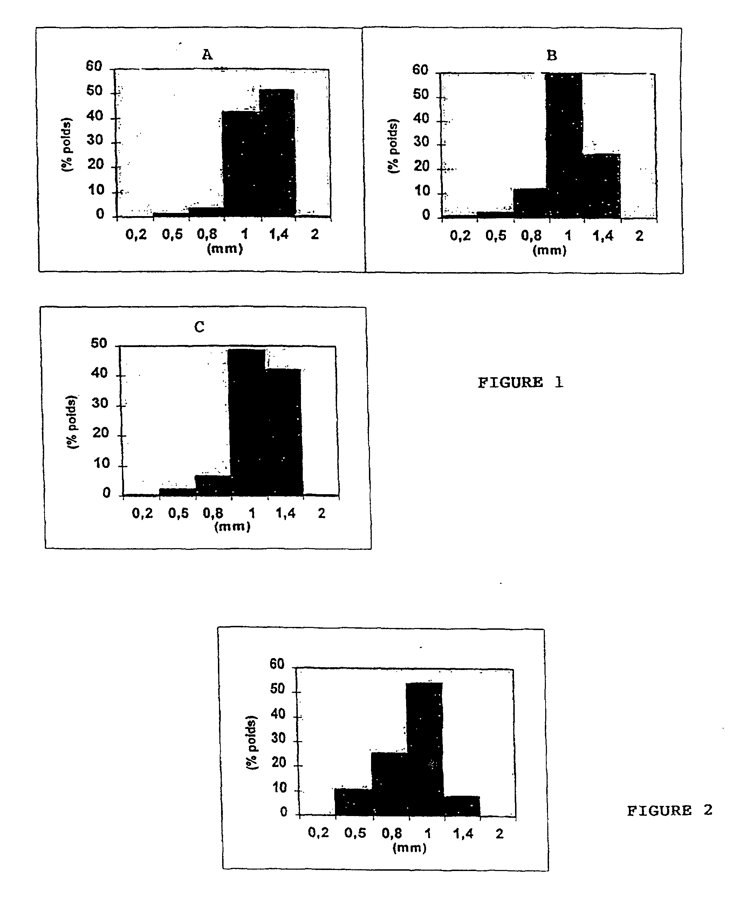

Dans ces conditions, on note qu'on obtient des pellets parfaitement individualisées présentant une distribution de taille pratiquement uniforme conformément à la figure 1.

Les pellets obtenus peuvent être ensuite incorporés dans des gélules ou des sachets.

Elles peuvent également être soumises à une étape de compression permettant d'obtenir des comprimés.

### Exemple 2

On répète l'exemple précédent en utilisant en tant qu'agent diluant le GELUCIRE® 50/13 du Demandeur.

On obtient dans les mêmes conditions de très belles pellets avec un temps d'agitation après fusion de 33 minutes. On obtient une bonne distribution des pellets conformément au graphique représenté en figure 2.

La présente invention présente donc un grand nombre d'avantages. Elle permet en effet de proposer des gélules du type SMEDDS dont le remplissage est effectué par un appareillage classique pour forme solide multiparticulaire.

En outre, les pellets de l'invention présentent l'avantage d'améliorer fortement la biodisponibilité du principe actif.

Enfin, le procédé de fabrication de ces pellets par pelletisation par fusion est rapide, simple à mettre en oeuvre, et peut être effectué dans un appareillage classique du type mélangeur à grande vitesse.

## Revendications

1. Pellet comprenant au moins une substance active, un agent liant et un agent diluant, susceptible d'être obtenue par le procédé selon lequel :
◆ tout d'abord, on introduit la substance active, l'agent liant et l'agent diluant dans un mélangeur, muni d'un système d'agitation planétaire et de chauffage, l'agent liant étant apte à améliorer la biodisponibilité de la substance active et :
• comprenant un mélange de glycérides polyglycolysés dont les acides gras comprennent au moins 8 atomes de carbone ;
• présentant un point de fusion supérieur ou égal à 37°C ;
• présentant une balance hydrophile/lipophile supérieure ou égale à 10;
◆ puis, on agite tout en chauffant les constituants jusqu'à atteindre une température proche du point de fusion de l'agent liant ;
◆ on poursuit l'agitation du mélange obtenu jusqu'à dispersion homogène de l'agent diluant et de la substance active dans l'agent liant ;
◆ on maintient l'agitation et la température afin de permettre l'agglomération, la densification et la sphéronisation des particules formées jusqu'à l'obtention de pellets individualisées ;
◆ toujours sous agitation et à une température sensiblement égale à celle du point de fusion de l'agent liant, on évacue les pellets individualisées en dehors du mélangeur ;
◆ enfin, on récupère les pellets et on les refroidit à une température permettant de solidifier et de maintenir les pellets individualisées, séparées les unes des autres.

2. Pellet selon la revendication 1, **caractérisée en ce que** le refroidissement des pellets est effectué sur des plateaux.

3. Pellet selon la revendication 1, **caractérisé en ce que** le refroidissement des pellets est effectué au moyen d'un lit d'air fluidisé.

4. Pellet selon l'une des revendications 1 à 3, **caractérisée en ce que** les pellets refroidis sont répartis dans des sachets ou des gélules.

5. Pellet selon l'une des revendications précédentes, **caractérisé en ce que** l'agent liant comprend un mélange de glycérides polyglycolysés saturés dont les acides gras comprennent de 8 à 18 atomes de carbones (C₈-C₁₈), présente un point de fusion égal à 44° C et une balance hydrophile/lipophile égale à 14.

6. Pellet selon l'une des revendications précédentes, **caractérisé en ce que** l'agent liant comprend un mélange de glycérides polyglycolysés avec des acides gras en C₂₂.

7. Pellet selon l'une des revendications précédentes, **caractérisée en ce que** la concentration d'agent liant représente entre 10 et 40 % en poids de la pellet.

8. Pellet selon l'une des revendications précédentes, **caractérisé en ce que** la concentration d'agent liant représente entre 15 et 20 % en poids de la pellet.

9. Pellet selon l'une des revendications précédentes, **caractérisé en ce que** l'agent liant peut comprendre en outre un additif choisi dans le groupe comprenant le polyéthylène glycol, les esters de glycérol, les esters de polyéthylène glycol, les esters de propylène glycol, les esters de polyglycérol avec des acides gras saturés et/ou insaturés, seuls en mélange.

10. Pellet selon l'une des revendications précédentes, **caractérisée en ce que** l'agent diluant se présente sous forme micronisée de taille comprise entre 1 et 300 micromètres.

11. Pellet selon l'une des revendications précédentes, **caractérisée en ce que** la substance active se présente sous forme micronisée de taille comprise entre 1 et 300 micromètres.

## Patentansprüche

1. Pellet, das wenigstens eine Wirksubstanz, ein Bindemittel und ein Verdünnungsmittel enthält und durch das Verfahren erhalten werden kann, gemäß dem:
◆ zunächst die Wirksubstanz, das Bindemittel und das Verdünnungsmittel in einen mit einem Planetenrührsystem und Heizung ausgestatteten Mischer gegeben werden, wobei das Bindemittel dazu geeignet ist, die biologische Verfügbarkeit der Wirksubstanz zu verbessern und:
• eine Mischung aus polyglykolisierten Glyceriden umfasst, deren Fettsäuren wenigstens 8 Kohlenstoffatome enthalten;
• einen Schmelzpunkt größer oder gleich 37°C aufweist;
• eine hydrophil-lipophile Balance größer oder gleich 10 aufweist;
◆ anschließend unter Erwärmen der Bestandteile bis zum Erreichen einer Temperatur nahe des Schmelzpunktes des Bindemittels gerührt wird;
◆ das Rühren der erhaltenen Mischung bis zur homogenen Verteilung des Verdünnungsmittels und der Wirksubstanz in dem Bindemittel fortgesetzt wird;
◆ das Rühren und die Temperatur aufrechterhalten werden, um das Agglomerieren, die Verdichtung und die Sphäronisierung der gebildeten Teilchen zu ermöglichen, bis zum Erhalt von individualisierten Pellets;
◆ unter weiterem Rühren und bei einer Temperatur im wesentlichen gleich der des Schmelzpunktes des Bindemittels die individualisierten Pellets aus dem Mischer ausgetragen werden;
◆ schließlich die Pellets wiedergewonnen und bei einer Temperatur abgekühlt werden, die es ermöglicht, die individualisierten, voneinander getrennten Pellets zu verfestigen und zu erhalten.

2. Pellet nach Anspruch 1, **dadurch gekennzeichnet, dass** das Abkühlen der Pellets auf Platten erfolgt.

3. Pellet nach Anspruch 1, **dadurch gekennzeichnet, dass** das Abkühlen der Pellets mittels eines Wirbelluftbetts erfolgt.

4. Pellet nach einem der Ansprüche 1 bis 3, **dadurch gekennzeichnet, dass** die abgekühlten Pellets in Beutelchen oder Kapseln verteilt werden.

5. Pellet nach einem der vorhergehenden Ansprüche, **dadurch gekennzeichnet, dass** das Bindemittel eine Mischung aus gesättigten polyglykolisierten Glyceriden enthält, deren Fettsäuren 8 bis 18 Kohlenstoffatome (C₈-C₁₈) enthalten, einen Schmelzpunkt gleich 44°C und eine hydrophil-lipophile Balance gleich 14 aufweist.

6. Pellet nach einem der vorhergehenden Ansprüche, **dadurch gekennzeichnet, dass** das Bindemittel eine Mischung aus polyglykolisierten Glyceriden mit C₂₂-Fettsäuren enthält.

7. Pellet nach einem der vorhergehenden Ansprüche, **dadurch gekennzeichnet, dass** die Bindemittelkonzentration zwischen 10 und 40 Gew. % des Pellets ausmacht.

8. Pellet nach einem der vorhergehenden Ansprüche, **dadurch gekennzeichnet, dass** die Bindemittelkonzentration zwischen 15 und 20 Gew.% des Pellets ausmacht.

9. Pellet nach einem der vorhergehenden Ansprüche, **dadurch gekennzeichnet, dass** das Bindemittel außerdem einen Zusatz ausgewählt aus der Gruppe enthaltend Polyethylenglykol, Glycerinester, Polyethylenglykolester, Propylenglykolester, Polyglycerinester mit gesättigten und/oder ungesättigten Fettsäuren, einzeln oder als Mischung, enthalten kann.

10. Pellet nach einem der vorhergehenden Ansprüche, **dadurch gekennzeichnet, dass** das Verdünnungsmittel in mikronisierter Form mit einer Größe zwischen 1 und 300 Mikrometern vorliegt.

11. Pellet nach einem der vorhergehenden Ansprüche, **dadurch gekennzeichnet, dass** die Wirksubstanz in mikronisierter Form mit einer Größe zwischen 1 und 300 Mikrometern vorliegt.

## Claims

1. Pellet comprising at least one active substance, one binder and one diluent which can be obtained by the process according to which:
◆ first of all, the active substance, the binder and the diluent are introduced into a mixer equipped with a planetary stirring and heating system, the binder being capable of improving the bioavailability of the active substance and:
• comprising a mixture of polyglycolysed glycerides, the fatty acids of which comprise at least 8 carbon atoms;
• exhibiting a melting point of greater than or equal to 37°C;
• exhibiting a hydrophilic-lipophilic balance of greater than or equal to 10;
◆ the constituents are then stirred, while heating, until a temperature close to the melting point of the binder is reached,
◆ the stirring of the mixture obtained is continued until the diluent and the active substance are homogeneously dispersed in the binder,
◆ the stirring and the temperature are maintained in order to make possible the agglomeration, the densification and the spheronization of the particles formed until individual pellets are obtained,
◆ the individual pellets are discharged from the mixer, still with stirring and at a temperature substantially equal to that of the melting point of the binder,
◆ finally, the pellets are recovered and are cooled to a temperature which makes it possible to solidify and to maintain the individual pellets separate from one another.

2. Pellet according to Claim 1, **characterized in that** the cooling of the pellets is carried out on trays.

3. Pellet according to Claim 1, **characterized in that** the cooling of the pellets is carried out by means of a fluidized air bed.

4. Pellet according to any one of Claims 1 to 3, **characterized in that** the cooled pellets are dispensed into sachets or hard gelatin capsules.

5. Pellet according to any one of the preceding Claims, **characterized in that** the binder comprises a mixture of saturated polyglycolysed glycerides, the fatty acids of which comprise from 8 to 18 carbon atoms (C₈-C₁₈), exhibits a melting point of 44°C and exhibits a hydrophilic-lipophilic balance of 14.

6. Pellet according to any one of the preceding Claims, **characterized in that** the binder comprises a mixture of polyglycolysed glycerides with C₂₂ fatty acids.

7. Pellet according to any one of the preceding Claims, **characterized in that** the concentration of binder represents between 10 and 40% by weight of the pellet.

8. Pellet according to any one of the preceding Claims, **characterized in that** the concentration of binder represents between 15 and 20% by weight of the pellet.

9. Pellet according to any one of the preceding Claims, **characterized in that** the binder can additionally comprise an additive chosen from the group consisting of polyethylene glycol, esters of glycerol, esters of polyethylene glycol, esters of propylene glycol and esters of polyglycerol with saturated and/or unsaturated fatty acids, alone or as a mixture.

10. Pellet according to any one of the preceding Claims , **characterized in that** the diluent is provided in the micronized form with a size of between 1 and 300 micrometres.

11. Pellet according to any one of the preceding Claims, **characterized in that** the active substance is provided in the micronized form with a size of between 1 and 300 micrometres.
